(19) Europäisches Patentamt / European Patent Office / Office européen des brevets

(11) **EP 4 725 396 A1**

(12) **EUROPEAN PATENT APPLICATION**

(43) Date of publication:
15.04.2026 Bulletin 2026/16

(21) Application number: 24205987.1

(22) Date of filing: 10.10.2024

(51) International Patent Classification (IPC):
$A61B\ 5/00^{(2006.01)}$     $G01B\ 9/02004^{(2022.01)}$
$G01B\ 9/02^{(2022.01)}$     $G01B\ 9/02055^{(2022.01)}$
$G01B\ 9/0209^{(2022.01)}$     $G01B\ 9/02091^{(2022.01)}$
$G01N\ 21/47^{(2006.01)}$

(52) Cooperative Patent Classification (CPC):
G01N 21/4795; A61B 5/0066; G01B 9/02004;
G01B 9/02044; G01B 9/02069; G01B 9/0209;
G01B 9/02091

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: **Comind Technologies Limited**
**London EC1R 5EJ (GB)**

(72) Inventor: **BORYCKI, Dawid**
**London, EC1R 5EJ (GB)**

(74) Representative: **Mathys & Squire**
**The Shard**
**32 London Bridge Street**
**London SE1 9SG (GB)**

(54) **METHOD AND APPARATUS**

(57) An aspect of the disclosure provides an apparatus comprising:
a reference channel for providing a reference light from a wavelength swept light source to a light combiner wherein the reference channel is configured to delay the reference light by a selected delay; a sample input channel for providing a sample input light from said wavelength swept light source and for directing the sample input light into a sample; a sample output channel for collecting, from the sample, a sample output light based on said sample input light and comprising a plurality of components each delayed, with respect to the sample input light, by a respective one of a plurality of delays; wherein the light combiner is configured to combine said sample output light with said delayed reference light to provide a combined light comprising a plurality of beat frequency components, each beat frequency component associated with a respective corresponding one of the plurality of delays; and, a controller configured with a predefined relationship for determining, from frequencies of the beat frequency components, a distribution of times-of-flight of light through the sample; wherein the predefined relationship is based on the selected delay provided by the reference channel being longer than any of the plurality of delays.

Figure. 1

**Description**

**Field of Invention**

The present invention relates to methods and apparatus for interferometry of a sample, such as a biological sample, and more particularly to methods and apparatus for swept source interferometry of samples which may comprise living tissue, such as brain tissue.

**Background**

Spectroscopy methods such as iNIRS, diffuse correlation spectroscopy ('DCS'), time-resolved DCS ('TD-DCS') or time-resolved NIRS ('TD-NIRS') can be used to infer properties of an object by directing light from a light source, such as a laser, towards that object and then using a detector to measure corresponding properties of the light received from that object. The received light may include some of the light which was directed towards the object from the light source, and which subsequently scattered from the object and towards the detector. By monitoring this received light over time, and how the received light signals may change, one or more properties of the object can be inferred. For example, GB2619063 discloses non-invasive intracranial pressure sensing systems and methods which utilise iNIRS for non-invasively determining the intracranial pressure of a subject.

One important parameter of in vivo sensing is the sensitivity as a function of the time-of-flight (TOF) of the photons through the sample. The longer the TOF the better the brain sensitivity and specificity because longer TOF normally indicates a deeper penetration (i.e., beyond the skin and skull).

Some existing methods have an unacceptably poor ability to detect photons with long TOF. Approaches to mitigate this include:

1. Improved Detector Design. Enhancing the detector's sensitivity can help it to detect weaker signals at longer TOFs. This means more expensive, higher-quality components.
2. Signal Processing Techniques. More sophisticated signal processing algorithms can help distinguish the actual signal from the noise, improving measurement accuracy. This comes at the cost of increased complexity.
3. Calibration and Compensation. Sensors can be calibrated to account for the decreased sensitivity at greater TOFs.

**Summary**

Aspects and examples of the present disclosure are set out in the claims and aim to address the above-described technical problem and other problems.

In an aspect there is provided an apparatus comprising:

a light combiner;
a reference channel for providing a reference light from a wavelength swept light source to the light combiner wherein the reference channel is configured to delay the reference light by a selected delay
a sample input channel for providing a sample input light from said wavelength swept light source and for directing the sample input light into a sample;
a sample output channel for collecting, from the sample, a sample output light based on said sample input light and comprising a plurality of components each delayed, with respect to the sample input light, by a respective one of a plurality of delays;
wherein the light combiner is configured to combine said sample output light with said delayed reference light to provide a combined light comprising a plurality of beat frequency components, each beat frequency component associated with a respective corresponding one of the delays; and,
a controller configured with a predefined relationship for determining, from frequencies of the beat frequency components, times-of-flight of light through the sample;

wherein the predefined relationship is based on the selected delay provided by the reference channel being longer than any of the plurality of delays.

The predefined relationship may be a monotonically decreasing relationship that associates beat frequency components of smaller frequency with longer times-of-flight and beat frequency components of greater frequency with shorter times-of-flight.

The apparatus may further comprise the wavelength swept light source, for example a laser, such as a tunable laser.

The apparatus may further comprise a light splitter configured to split light from said wavelength swept light source to provide the reference light and the sample input light.

The apparatus may further comprise a variable delay line between one of:

(i) said light source and said sample and
(ii) said sample and said light combiner.

The controller may be configured to control the variable delay line to adjust said delays of said sample output light, wherein the adjusting is based on frequencies of the beat frequency components.

The apparatus may further comprise a variable delay line in said reference channel.

The controller may be configured to control the variable delay line to adjust the selected delay based on frequencies of the beat frequency components.

In an aspect there is provided an apparatus configured to determine times-of-flight of light through a sample up to a maximum time-of-flight associated with a maximum delay of interest, the apparatus comprising a controller configured to:

obtain input data from a light detector, wherein the input data defines a combined light signal obtained from interference of a sample output light with a delayed reference light;
determine, from the input data, frequency data defining beat frequencies of components of the input data;
determine a plurality of times-of-flight from the frequency data based on a predefined relationship associating lower beat frequencies with longer times-of-flight through the sample and higher beat frequencies with shorter times-of-flight through the sample;
wherein the sample output light comprises a plurality of light components collected from a sample and based on sample input light provided into the sample and the delayed reference light corresponds to the sample input light delayed by a delay greater than the maximum delay of interest.

In an aspect there is provided a computer implemented method of determining times-of-flight of light through a sample up to a maximum time-of-flight associated with a maximum delay of interest, the method comprising:

obtaining input data from a light detector, wherein the input data defines a combined light signal obtained from interference of a sample output light with a delayed reference light;
determining, from the input data, frequency data defining beat frequencies of components of the input data;
determining a plurality of times-of-flight from the frequency data based on a predefined relationship associating lower beat frequencies with longer times-of-flight through the sample and higher beat frequencies with shorter times-of-flight through the sample;
wherein the sample output light comprises a plurality of light components collected from a sample and based on sample input light provided into the sample and the delayed reference light corresponds to the sample input light delayed by a delay greater than the maximum delay of interest.

In an aspect there is provided a method of determining, from a combined light signal, times-of-flight of light through a sample corresponding to a plurality of delays of the combined light signal, the plurality of delays comprising delays up to a maximum delay of interest, the method comprising:

receiving, at a detector, the combined light signal wherein the combined light signal is based on interference of a sample output light with a delayed reference light;
wherein the sample output light is based on sample input light provided into the sample;
the combined light signal comprising a plurality of beat frequency components, each beat frequency component associated with a corresponding one of a plurality of delays of said sample output light with respect to the sample input light,
wherein said delayed reference light corresponds to the sample input light delayed by a delay greater than the maximum delay of interest; and,
determining times-of-flight of light through the sample based on frequencies of the beat frequency components.

In an aspect there is provided a spectrography system, said system comprising:

a light source configured to provide a reference light and a sample input light;
a sampling arrangement configured to direct the sample input light into a sample and to receive a sample output light comprising components based on said sample input light;
a light combining apparatus configured to combine said sample output light with said reference light to provide a combined light comprising a set of beat frequencies based on a set of delays of said sample output light from said light source to said light combining apparatus;

wherein said set of delays of said sample output light comprises a maximum delay of interest y;

wherein a delay of said reference light from said light source to said light combining apparatus is x and x>y.

The light source may be made and sold separately. The sampling arrangement may comprise a sample delivery probe and a sample receiving probe as described herein. The light combining apparatus may comprise a light combiner.

In an aspect there is provided a method of determining a time-of-flight of light through a sample, the method comprising:

collecting, from the sample, a sample output light based on sample input light provided into the sample, wherein interaction of the sample input light with the sample provides a plurality of respective delays of the sample output light with respect to the sample input light, and said delays comprising delays less than or equal to a maximum delay of interest,

combining said sample output light with a delayed reference light to provide a combined light comprising a plurality of beat frequency components, each beat frequency component being associated with a corresponding one of the delays of the sample output light,

wherein said delayed reference light corresponds to the sample input light delayed by a delay greater than the maximum delay of interest; and,

determining times-of-flight of light through the sample based on frequencies of the beat frequency components.

Delaying the reference light by a delay greater than the maximum delay of interest provides a configuration of the combined light signal in which smaller beat frequencies are associated with longer times-of-flight in the sample (greater delay associated with the sample).

This is significant where there is a need to detect long times-of-flight. For example, where the sample comprises tissue, long times-of-flight may be associated with deeper tissue penetration. In measurements of the human brain, the photons may need to penetrate scalp, skull, and meninges before interacting with brain tissue. Accordingly, photons with deep penetration may be of particular interest. One important parameter of sensing is the sensitivity as a function of the time-of-flight (TOF) of the photons through the sample. In prior systems, longer TOF indicates a deeper penetration and long TOFs are typically encoded as high beat frequencies. This is a problem because of the following considerations:

1. Electronic detection bandwidth - a detector's sensitivity may decrease with increasing beat frequency. Thus, photons with a long TOF are harder to detect because of reduced sensitivity.

2. Dynamic coherence time - Longer TOFs lead to decreasing coherence between the reference light and the received light. The reduced coherence means that random phase fluctuation noise increases. The increased phase fluctuation noise reduces the recoverable amplitude of the beat frequencies.

High-bandwidth detectors typically have low internal gain, which results in low signal amplitudes and poor signal-to-noise ratio. By associating long TOF (and greater penetration depth) with lower frequencies, the present disclosure addresses these problems permitting lower bandwidth detectors and slower sampling electronics.

Embodiments of the present disclosure may therefore address the problem of limited electronic detection bandwidth and potentially improve dynamic coherence time to enhance the system's efficiency in detecting late TOF signals.

The times-of-flight through the sample may be determined by using a predefined relationship such as that described above. This relationship may be configured into the apparatus of the present disclosure, such as the controllers described herein. The relationship may comprise associating smaller frequencies of the beat frequency components with shorter delays and greater frequencies of the beat frequency components with longer delays. The frequencies may be negative - so by "lower" it is meant "of lesser magnitude" and by "higher" it is meant "of greater magnitude". The times-of-flight may be determined based on a predefined relationship which maps beat frequencies to times-of-flight and associates progressively higher beat frequencies with progressively shorter times-of-flight. For example, this predefined relationship may be monotonically decreasing.

The wavelength swept light may be provided from a laser, such as a tunable laser.

Providing the reference light and the sample input light may comprise splitting the wavelength swept light using a beam splitter or a fibre optic coupler.

The method may comprise adjusting said delays of said sample output light using a variable delay line between one of:

(i) said light source and said sample and

(ii) said sample and said light combining apparatus.

The method may comprise adaptively adjusting said delays, for example based on frequencies of the beat frequency components.

The method may comprise adjusting the delay, x, of the reference light, for example wherein said delay x is adjusted by controlling a variable delay line between the light source and the light combiner. The method may comprise adaptively adjusting the delay, x, for example adaptively adjusting said delay, x, based on frequencies of the beat frequency components.

The variable delay line may comprise a fibre length or a free-space delay line, for example.

The maximum delay of interest y may be selected from said plurality of delays based on identifying a beat frequency component using a power or amplitude threshold.

The computer implemented data processing methods described herein may further comprise operating the light detector to detect the combined light and providing input data for the data processing method based on said detecting.

Embodiments may comprise operating a wavelength swept light source of a spectrographic measurement system to provide the reference light and the sample input light.

Embodiments may comprise controlling at least one variable delay line to provide an adjustable delay of at least one of the reference light and the sample input light, for example

wherein the adjustable delay is determined adaptively based on frequencies of the beat frequency components.

Embodiments provide a computer program product configured to program a programmable processor to perform any one or more of the methods described and claimed herein.

Such a processor may further comprise or be coupled to a communication interface for obtaining the input data from the detector and/or for controlling operation of the light source and/or for controlling operation of a variable delay line.

An aspect of the disclosure provides an apparatus configured to perform any one or more of the methods described and claimed herein.

The maximum delay of interest may be a delay associated with an path length in biological tissue or an anatomical structure. The biological tissue may comprise human brain tissue and/or the anatomical structure may comprise part of the human head comprising at least part of the scalp and the skull. The maximum delay of interest may be selected based on a centre wavelength of a light source of the apparatus.

Other embodiments are envisaged.

## Brief Description of Drawings

Embodiments of the disclosure will now be described in detail with reference to the accompanying drawings, in which:

Figure 1 shows a diagram of an apparatus according to the present disclosure;
Figure 2 shows a flow chart illustrating a method of determining times-of-flight of light through a sample;
Figure 3 shows a flow chart of a data processing method;
Figure 4 shows a flow chart illustrating how to configure an apparatus such as that illustrated in Figure 1;
Figure 5 shows some illustrative plots indicating simulated signals corresponding to those obtained in apparatus according to the present disclosure; and
Figure 6 shows a flow chart illustrating a method of determining the maximum delay of interest for which an apparatus according to the present disclosure is configured.

In the drawings like reference numerals are used to indicate like elements.

## Specific Description

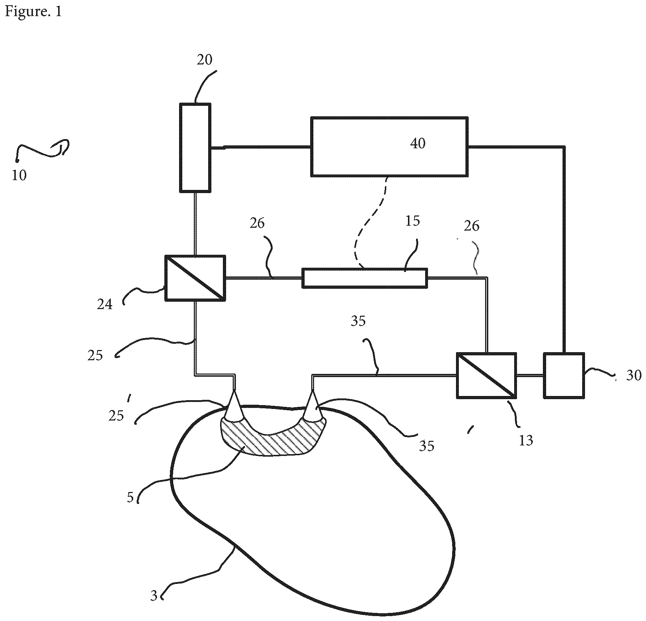

An example of a spectrographic apparatus for measurement of a sample will now be described with reference to an interferometric Near Infrared Spectroscopy ('iNIRS') system, as shown in Figure 1.

The apparatus 10 includes a light source 20, a light splitter 24, a sample delivery probe 25', a sample receiving probe 35', a delay line 15, a light combiner 13, a detector 30, and a controller 40. The apparatus also 10 includes a sample delivery channel 25, a reference delivery channel 26, and a sample output channel 35. There is also shown a sample 3 and a volume of the sample 5, as described below.

The light source 20 is arranged to provide light to the light splitter 24.

The light splitter 24 is connected to the sample delivery probe 25' by the sample delivery channel 25. The light splitter 24 is also connected to light combiner by the reference delivery channel 35. The reference delivery channel 26 may comprise the delay line 15, or the delay line may otherwise be interposed in the connection between the light splitter 24 and the light combiner 13.

As mentioned above, also shown in Figure 1 is a sample 3, and the sample delivery probe 25' and the sample receiving probe 35' are shown as being in contact with the sample 3. Also indicated in Figure 1 is a volume of the sample 5, disposed between the sample delivery probe 25' and the sample receiving probe 35'.

In the arrangement illustrated in Figure 1, the sample delivery probe 25' is arranged for providing light, received along the sample delivery channel 25 into the sample 3. This light, which is provided to the sample 3 by the sample delivery probe 25' may be referred to herein as the sample input light.

In addition, the arrangement illustrated in Figure 1 also shows the sample receiving probe 25' arranged for receiving light from the sample. This light, which emerges from the sample as a result of the delivery of the sample input light to the sample 3, may be referred to herein as the sample output light.

Photons of sample input which enter the sample 3 may undergo a large number of scattering events between the sample delivery probe 25' and the sample receiving probe 35'. A photon which travels a longer path, and so is subject to a greater delay is likely to have penetrated more deeply into the sample 3. The volume of sample with which the photons may interact is indicated generally by the hatched area shown in Figure 1. It will be appreciated that this illustration is merely schematic.

The light source 20 may comprise a laser, such as a wavelength swept laser, which may also be referred to as a tunable laser. For example, the light source may comprise a Distributed Feedback laser ('DFB') or a MEMS-Vertical Cavity Surface Emitting laser ('MEMS-VCSEL').

The light splitter 24 is configured to divide light provided to it by the light source between a) the reference delivery channel and b) the sample delivery channel. The light provided to the reference delivery channel 26 may be referred to herein as the reference light, because it provides a phase reference for the sample input light. The light splitter 24 may comprise a beam splitter - examples include plate beam splitters and cube beam splitters.

The sample receiving probe 35' is connected to the light combiner 13 by the sample delivery channel 35. The sample receiving probe 35' is also configured to provide the sample output light, via the sample delivery channel 35, to the light combiner 13.

The delay line 15 is configured to delay the reference light by a selected amount. The delay line 15 and the reference delivery channel 26 together arranged to deliver delayed reference light to the light combiner 13. The delay of the reference light may be preconfigured - for example it may be set by the optical path length of the delay line 15. The delay may be preconfigured for operation with a predefined range of times-of-flight in the sample 3. Typically, the delay line 15 is configured to apply a delay sufficient that substantially all times-of-flight in the sample (i.e. delays caused by the sample to the sample output light with respect to the sample input light) are less than the delay applied to the reference light.

When the delay line 15 is configured in this way then the observed "delays" of the sample output light are predominantly negative - in the sense that the sample output light arrives, at the light combiner, ahead of the corresponding delayed reference light. For example, the delays of the sample output light being "predominantly" negative may mean that the intrinsic delay provided to the reference light by the delay line may be such that the signal power associated with sample output light delayed by more than that intrinsic delay is much less than the signal power of less delayed light with which it might "overlap" in the interferogram. This might be understood as substantially all of the observed delays being negative. The amount by which it will be ahead is set by the difference between (a) the delay imposed by the reference delivery channel 26 and delay line; and (b) the delay due to time-of-flight in the sample 3.

In some embodiments the delay caused by the delay line 15 may be adjustable, and the controller may be connected to control the delay line 15 to adjust the delay which it applies to the reference light The delay line may be, for example, a free-space or in-fibre optical delay line. Examples of suitable delay lines include the ODL100(/M) Optical Delay Line series of instruments and kits which are available from Thorlabs Ltd of 204 Lancaster Way Business Park, Ely CB6 3NX, United Kingdom.

The light combiner 13 is arranged to receive delayed reference light from the reference delivery channel 26 and to combine it with light received from the sample output channel 35 to provide a combined light signal. Where there is sufficient coherence between the two, the combined light signal may exhibit the effects of interference between the sample output light and the delayed reference light.

The light combiner 13 is configured to provide the combined light signal to the detector 30.

The detector 30 may comprise any appropriate light detector, such as an image sensor or photodetector. The detector 30 is connected to the controller for providing signals to the controller 40 indicating an intensity of the combined light signal provided to the detector. These signals may be provided as a time series of data, whether in analogue or digital form, representing the intensity of the combined light signal at the detector 30 as a function of time.

It will be appreciated in the context of the present disclosure that, using a cosine series, the light incident on the detector may be written as:

$$I = \sum_i a_i cos[k(l_i - \Delta z)],$$

where $k=2\pi/\lambda$ is the wave number of the combined light signal, $\Delta z=z_s-z_r$ is the interferometer path mismatch with $z_r$ and $z_s$ denoting the lengths of the reference and sample arm, respectively. The coefficient $a_i$ indicates an amplitude of a particular cosine component component, $l_i$ is the "path length" associated with that cosine component (the product of the time-of-flight TOF in the sample and the speed of light, $c - c^*TOF$). It will be appreciated in the context of the present disclosure that $a_i$ may indicate a probability of detecting a photon that propagated the path of length $l_i$ inside the sample.

The controller 40 may perform a frequency transform (such as a Fourier transform) of the signal, I, from the detector, which may be written as:

$$FT_{k->l}\{I(k)\} = \pi \sum_i a_i\{\delta(l - l_i - \Delta z) + \delta(l + l_i + \Delta z)\}$$

where $\delta(l)$ denotes a Dirac delta function centred at $1$. It can therefore be seen that the frequency transform performed by the controller results in signal components having amplitude $a_i$ at frequencies (in the transformed signal) which correspond to path length differences $l=l_i- \Delta z$ and at $l=l_i+ \Delta z$. In the simplest case of a single delay (single path through the sample) $l_1$, this provides a delta function of amplitude $a_1$. It can also be seen that the

Fourier transform (as a Fourier transform of a real signal) has Hermitean symmetry [i.e., F*(x) = F(-x)].

The frequency components of the combined light signal may be referred to herein as "beat frequencies" because they arise as "beats" caused by interference between coherent light of slightly mismatched wavelength arriving at the detector. The mismatch in wavelength arising from the combined effect of the wavelength sweep of the sample input light and reference light, respectively. The controller may be configured with a predefined relationship for transforming the beat frequency components of the measured signal into time-of-flight data. For example, this predefined relationship may associate lower beat frequency components with longer path lengths in the sample and higher beat frequencies with shorter path lengths in the sample. For example, the predefined relationship may be based on $\Delta z$ - i.e. the path length difference between:

(a) $z_r$ the optical path length from the light splitter to the beam combiner for the delayed reference light; and
(b) $z_s$ the optical path length from the light splitter to the beam combiner for the sample output light (not including path length in the sample itself).

Because the delay line is configured as described above, for substantially all delays caused by the sample (having sample path lengths $l_i$, $z_s+l_i<z_r$ so the respective delays are "mirrored" around the zero delay line. As a result, smaller timing differences between sample output light and delayed reference light are associated with longer path length in the tissue. This means a lower frequency for the longer path lengths.

This is significant because it may reduce the optical path length difference associated with long times-of-flight (and so reduce random phase fluctuation noise) it may also reduce the electronic detection bandwidth required to sense the beat frequencies associated with long times-of-flight.

It will be appreciated by the skilled addressee, having read the present disclosure, that the predefined relationship is based on an assumption that the selected delay (provided in the reference arm) is longer than any of the plurality of delays. This does not require that the selected delay *actually is* longer than every delay caused by the sample. Instead, this merely means that the controller is configured with a relationship which is based on the assumption that this is the case. The "selected delay" parameter is a feature of the configuration of the controller and of the reference arm (and any variable delay line associated with it) rather than any property of the sample. Some of the delays caused by the sample may of course be longer.

In practice, this is an acceptable assumption even if it is not perfectly correct because, as will be explained below, where the selected delay applied in the reference line is sufficiently long as to be longer than the delays associated with most of the signal power, then the frequencies of the beat frequency components are "flipped" or reflected about a notional zero frequency. This may be thought of as the timing offset between the sample arm and the reference arm of the interferometer being such that the reference light arrives at the light combiner far enough ahead of the corresponding sample light that the measured "delay" associated with transport through the sample is negative for most of the signal power in the combined signal.

If, in any particular case of using the apparatus, it is determined that the above assumption is too badly wrong and too much signal power is associated with sample delays that are shorter than the selected delay applied in the reference arm, then the delay applied by the reference arm can be increased by the variable delay line and a corresponding adjustment made to the predefined relationship used to calculate times of flight.

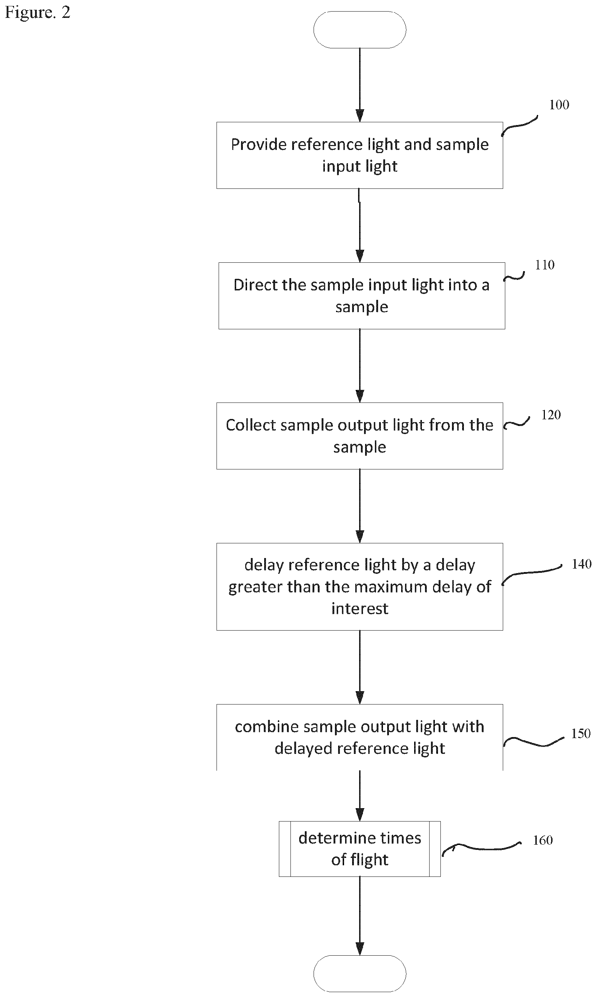

Operation of apparatus 10 such as that shown in Figure 1, to determine a distribution of times-of-flight of light through the sample will now be described with reference to Figure 2.

A wavelength swept light, such as a laser beam, is provided 100 from the light source to the light splitter which splits it to provide, on the reference delivery channel, the reference light and, on the sample delivery channel, the sample input light.

The sample input light is directed 110 to the sample delivery probe by the sample delivery channel. The sample delivery probe then couples this light into the sample. This provides the sample input light into the sample.

The reference light's journey along the reference delivery channel is delayed by the delay line. The delay applied by the delay line is selected according to the maximum delay (maximum time-of-flight in the sample) which is of interest in the measurement. The delayed reference light is carried to the light combiner by the reference delivery channel.

The sample input light travels through the sample, from the sample delivery probe to the sample receiving probe. During this journey, different photons are scattered differently and interact differently with different parts of the sample. Some photons may penetrate very deeply while other may not penetrate much at all. The sample output light, which emerges from the sample due to its illumination by the sample, is collected 120 by the sample receiving probe. The sample delivery channel carries the sample output light to the light combiner.

The result of the multiple paths which may be taken by photons through the sample is that, rather than there being a single time-of-flight instead there exists a distribution of times-of flight of the photons. Expected properties for a time-of-flight distribution for sample light may be known or estimated *a priori.* For instance, a shortest time-of-flight for photons may be known and an expected longest time-of-flight may be known or at least estimated. Such an estimate may be based on knowledge of the sample and/or observations and testing of the same or similar samples. As there is a distribution of times-of-flight, there is also a distribution of delays of photons as they exit the sample. The sample output light thus comprises parts which have been delayed by different amounts according to the different interactions with the tissue. The sample output light, including these parts with a distribution of different delays, is provided to the light combiner which combines 150 them into a single combined light signal.

In the combined light signal interference may arise between the delayed reference light and the sample output light.

The detector detects the intensity of the combined light signal and provides a signal time course (e.g., such as a series of digital samples) defining the intensity of the combined light signal incident on the detector.

The controller determines 160 times-of-flight of the light through the sample by performing a frequency transform of this data. The frequency transform provides an indication of signal amplitude at a plurality of beat frequencies. Each of these signal amplitudes relates to the amplitude, $a_i$, of a corresponding beat frequency component in the combined light signal. The controller may use a predefined relationship (e.g. a relationship stored in memory) associating beat frequencies with times-of-flight to determine the time-of-flight distribution of the light through the sample. For example, because $I = \sum_i a_i cos[k(l_i - \Delta z)]$, the cosine argument / frequency may increase with path length $l_i$ of path i for a given $\Delta z$.

It may be noted that the frequency dependency of the cosine depends on the difference $\Delta z$, i.e., $\Delta z = z_s - z_r$. Thus, the absolute values of $z_s$ and $z_r$ may be unimportant. Correspondingly, a delay, such as delay line 15, may be used to modify either $z_s$ or $z_r$.

The signal provided from the detector representing the time course of the combined light signal may be provided as an output in its own right, for example it may be stored in a memory or transmitted as a network message or otherwise output to a resource. This signal from the detector may be processed independently to determine characteristics of the sample, such as the time-of-flight distribution. For example, the optical measurement of the sample may be performed in one location and the analysis of the data might be performed remotely or at a later time. To this end, the controller operable to process the time course data may not need to be part of data acquisition part of the system.

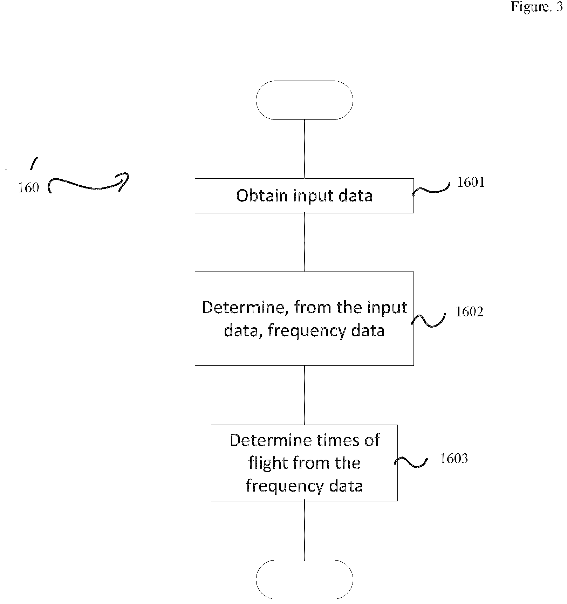

The process for determining times-of-flight from this signal 160' will now be described with reference to Figure 3.

The controller first obtains 1601 the input data, such as from the detector or from another resource to which it has been provided.

The input data defines the combined light signal obtained from interference of a sample output light with a delayed reference light, as described above with reference to Figure 1 and Figure 2.

The controller then performs a frequency transformation, such as a Fourier transform, of the input data to determine 1602 frequency data. The frequency data comprises coefficients, $a_i$, each indicating the amplitude of a corresponding frequency component of the input data. These correspond to the beat frequency components of the combined light signal which was used to generate the input data.

The controller then determines, for each of these frequencies, an associated time-of-flight. These times-of-flight may be determined from a predefined relationship which is based on data indicating the delay applied by the delay line. This delay provides an upper limit on the maximum delay of interest which may be determined from the data, for example.

The predefined relationship may comprise a mapping from beat frequencies to times-of-flight, which maps low beat frequencies to long times-of-flight and progressively higher beat frequencies to progressively longer times-of-flight.

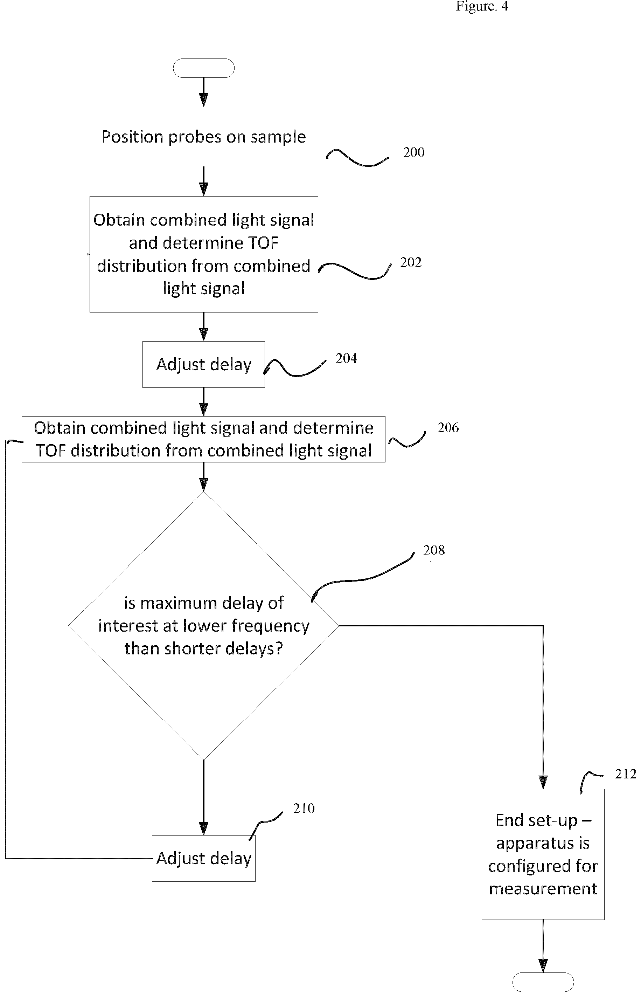

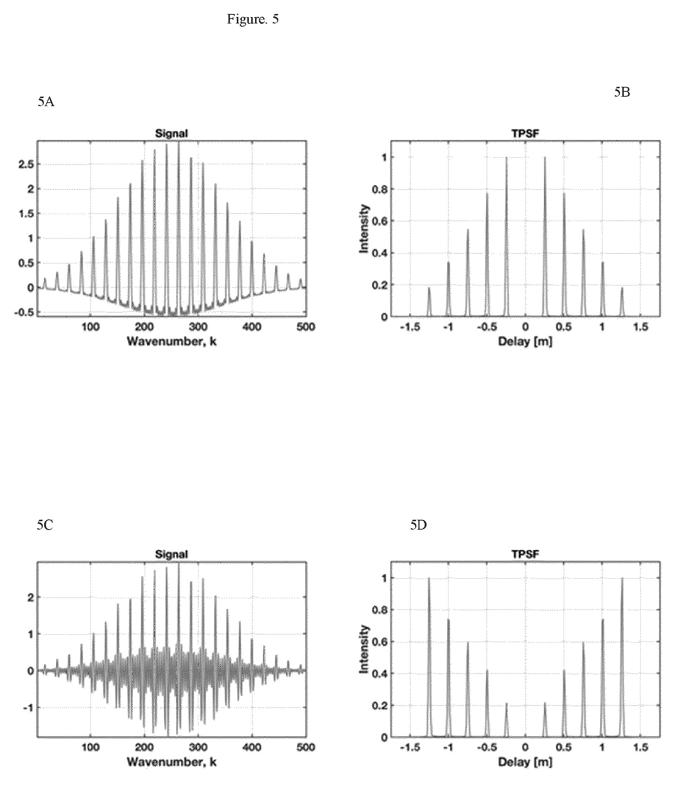

An example of how to configure an apparatus such as that described with reference to Figure 1 will now be described with reference to Figure 4 and Figure 5.

First, the sample delivery probe and the sample receiving probe are positioned 200 on a sample and the combined light signal is generated as described above but without the delay line 15 in the reference delivery channel 26. In this configuration, the path length of the reference delivery channel and the sample arm of the apparatus (to the probes and onward to the light combiner) may be substantially equal, or offset by some small predefined amount, so that time-of-flight in the sample delays the sample output light with respect to the reference light.

The combined light signal is detected 202 and provided to the controller 40. An illustration of an interferogram associated with such a measurement is shown in Figure 5A. The controller then performs a frequency transform of this data to generate an indication of the beat frequency components. An illustration of these is shown in Figure 5B. It can be seen that a distribution of beat frequencies is obtained. In this example, the higher signal power is associated with shorter delays. A maximum delay of interest may be estimated or decided based on such a measurement. It will be appreciated in the context of the present disclosure that this step of measuring the times-of-flight initially without delaying the reference light to provide "negative delay" of the sample output light is optional, and it may be possible to select an intrinsic delay for the reference light without performing this step.

A delay line may then be introduced to add 204 a delay to the reference signal, and the process of obtaining an interferogram and calculating the times-of-flight distribution may be repeated 206. It is then determined 208 whether the delay introduced by the delay line is appropriate. This may be assessed based on the area under the time-of-flight distribution. When the delay is appropriate, a predominant amount of the delays provided by the sample are less than the delay introduced by the delay line, the frequency transform of the interferogram may be reversed, as compared to the prior frequency transform (compare Figure 5B and Figure 5D).It can be seen from comparison of Figure 5A and Figure 5C that, where the device-intrinsic delay is appropriately large to "flip" the TOF distribution as illustrated in Figure 5D, then the interferogram signal may comprise higher signal power in the higher frequencies.

If the delay is small (and not sufficient to "flip" the TOF distribution enough), then the distribution may be "folded" so that it overlaps with itself because some of the apparent delays are positive and some are negative. This may be observed as an overlap between the apparent positive delays and negative delays in the transformed interferogram. In the event that such overlap is present, the delay may be increased 210 and the interferogram obtained again with the new delay. The process 206, 208, 210 of checking for this overlap and increasing the delay may be repeated until the delay provided by the delay line allows the distribution of times-of-flight from the sample to be observed mostly (e.g. predominantly) as negative delays (with the two halves of the distribution separated).

Once the delay is appropriately long, short delays are represented by small beat frequencies. The apparatus configured in this way 212 may be used to obtain better measurements. Experiments performed by the inventors have shown that at smaller beat frequencies the detector provides better gain and better coherence and thus less random phase noise. In other words, the long TOFs with smaller amplitudes can now benefit from the better sensitivity of the detectors.

The maximum delay of interest may correspond to a path length difference of 20 cm, or up to 200 cm. For a wavelength of 1064nm, the path length difference maximum delay of interest may correspond to a path length difference of 50 cm to 1.60 cm.

To explain how to adjust the optical delay line, we start with the expression for the maximum sensing range of the interferometer, $z_{max}$ :

$$z_{max} = \frac{\pi N}{2 \Delta k},$$

where:

- $N = \frac{f_s}{f_l}$  is the number of samples in a wavelength sweep of the wavelength swept light source,
- $f_s$ is the sample rate (e.g. 100 MHz),
- $f_l$ is the laser sweep rate (e.g. 100 kHz),

- $\Delta k = \frac{2\pi}{\lambda_{end}} - \frac{2\pi}{\lambda_{start}}$  is the laser sweep bandwidth, given in wavenumber.

If the optical mismatch between two interferometer arms is $\Delta L$, the TPSF will mostly start not earlier than $\Delta L$. If we want to flip the TPSF so that it can reliably be detected in negative delays, we need to add an extra delay $\Delta L' = z_{max}$. Hence, the range of additional delay will be between 0 and $z_{max}$.

Assuming the centre wavelength of 1064 nm, the tuning bandwidth of 0.2 nm, and N=1000, we get $\Delta k = 1.13 \times$

$10^3$. This leads to a $Z_{max}$ of 1.42 m. However, 1064 nm wavelength is just an example, and the present disclosure may be used for other wavelengths too. Some examples of appropriate values are listed in the Table below, but other values may be used.

| $\lambda_c$ [m] | $\Delta\lambda$ [m] | $\Delta k$ [m] | N | $z_{max}$ [m] |
|---|---|---|---|---|
| 1064 | 0.2 | 1 110.01 | 1 000 | 1.42 |
| 1064 | 0.2 | 1 110.01 | 500 | 0.71 |
| 850 | 0.4 | 3 478.58 | 1 000 | 0.45 |
| 850 | 0.4 | 3 478.58 | 500 | 0.23 |
| 850 | 0.2 | 1 739.29 | 1 000 | 0.90 |
| 850 | 0.1 | 869.65 | 1 000 | 1.81 |
| 780 | 0.1 | 1 032.74 | 1 000 | 1.52 |

The maximum delay of interest may be selected according to the centre wavelength and/or the sweep rate of the wavelength swept light source. The maximum delay of interest may correspond to a selected path length, which may be predefined. For example, the method or apparatus of the present disclosure may be configured with a maximum delay of interest, which may correspond to at least 0.2 m, and, for example, less than 2m.

The centre wavelength of the light source may comprise 1064nm and in these embodiments the maximum delay of interest may correspond to at least 0.5 m, for example 0.7 m, and for example less than 1.6m, for example less than 1.5m or for example less than 1.42m.

The centre wavelength of the light source may comprise 850nm and in these embodiments the maximum delay of interest may correspond to at least 0.2 m, for example less than 2m, for example less than 1.81m.

It will also be appreciated that by the method disclosed above, for any given sample, it is possible to determine what length of delay should be introduced by the delay line to obtain the benefits of the present disclosure. It will also be appreciated that different delays may be appropriate for different samples and different materials or tissues.

Presented with an apparatus having the structure explained with reference to Figure 1, it is also possible to determine the "maximum delay of interest" for which that apparatus is configured. It will be appreciated in the context of the present disclosure that this parameter of the apparatus may be adjustable. In the event that it is adjusted (for example by changing a delay applied by a variable delay line, as described elsewhere herein), a corresponding adjustment may be made to the predefined relationship used by the controller to determine the time-of-flight distribution from the frequencies of the beat frequency components.

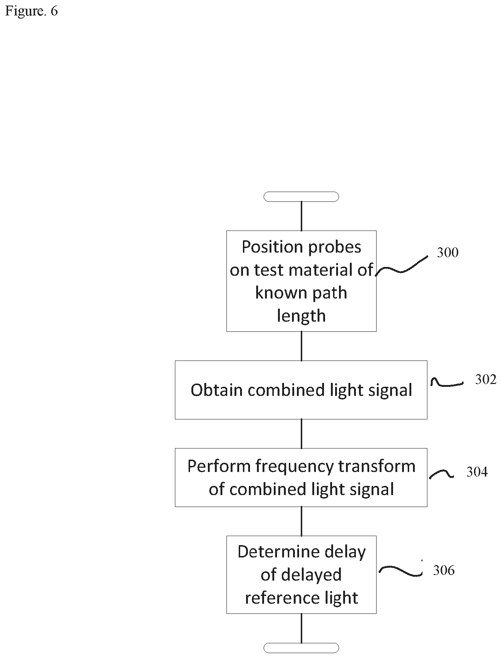

As illustrated in Figure 6, such a method may comprise configuring 300 the sample receiving probe and the sample delivery probe. For example, such a configuration may comprise optically short-circuiting the sample delivery probe and the sample receiving probe. Then, the lengths of the paths are known because the lengths of the fibres and other optical elements (e.g., delay lines) are known. Without a sample in the sample light path, the random path length normally introduced by the sample is not present.

The combined light signal can then be obtained 302 and the interferogram frequency transformed 304 to determine 306 the delay associated with the sample of known path length. From this measurement, the delay introduced by the device and measurement setup can be directly inferred. This may be referred to as the device-intrinsic delay.

In embodiments of the disclosure typically this device intrinsic delay is set to be greater than any delay expected to be introduced by the samples for which the device is to be used. This is described above as the delay applied to the reference light and is defined in the appended claims as the "selected delay". The method described above may be used to identify the device intrinsic delay and so to assess the maximum times-of-flight in a sample for which the apparatus may be used to implement the methods of the present disclosure. It will be understood by the person skilled in the art, that by introducing variable delays into the reference light path and/or into the sample light path, the apparatus and/or method can be adjusted for any smaller times-of-flight in a sample.

The controller of apparatus according to the present disclosure may be configured with a predefined relationship which is based on this selected delay so that any time-of flight in a sample (any medium optically coupling the sample delivery probe to the sample receiving probe) which is less than the selected delay is manifest as a "negative" beat frequency.

It will therefore be appreciated that, when presented with any apparatus according to the present disclosure, it is possible to directly and positively verify whether the device-intrinsic delay (e.g. the delay applied to the reference light) corresponds to the predefined relationship which is used by the controller to determine the times-of-flight from the beat frequencies of the interferogram.

Other embodiments are envisaged.

The present disclosure has been explained with reference to samples in general but may find particular uses in observation of biological samples, such as living human tissue, for example for in-vivo measurements. In such examples, the maximum delay of interest may be selected based on a *priori* knowledge, such as the anatomy of the part of the human or animal body which is to be used as a sample. As one example, the disclosure may provide an apparatus for performing measurements of the human or animal brain. The maximum delay of interest may be selected based on the penetration depth needed to provide interaction between the sample light and the brain tissue. Measurements of the distribution of times-of-flight of light of different wavelengths may permit measurement of intracranial pressure and so forth. These measurements may be performed based on determining differential absorption between different frequency bands and at different penetration depths. For example, the light source 20 may be configured to sweep in optical frequency over a range of 50 GHz. For example, this may enable the light source 20 to emit light at a plurality of different wavelengths between e.g., 829.94 nm and 830.06 nm when centred on 830nm for example or between 1309.857 nm and 1310.143 nm when centred on 1310 nm for example. The light source 20 may be configured to sweep through a wavelength range of at least 0.025 nm, such as at least 0.05 nm, such as at least 0.075 nm, such as at least 0.1 nm, such as at least 0.11 nm (e.g., about a wavelength on which it is centred).

Other physiological measurements may also be performed.

The sample delivery channel and the reference delivery channel may comprise any appropriate optical components such as lenses, collimators, waveguides such as optic fibres and so forth. Any appropriate means of conveying the source light to the relevant components of the system may be used.

The sample delivery probe and sample receiving probe may comprise surfaces suitable for optical coupling with a sample for example the surface of the probe may be suitable for optical coupling with a living human or animal body such as by optical coupling with the skin. The probes may comprise lenses, such as collimating lenses and other appropriate elements. Such a surface may be provided by the end of an optic fibre or an optic fibre coupling.

The light combiner may be provided by a half-silvered mirror, a fibre-coupler, a beam splitter of any appropriate type or other similar structure.

The wavelength swept light described herein may be provided as a beam. It will therefore be appreciated that the reference light, delayed reference light, sample input light, sample output light, and combined light signal may each accordingly also be beams. It will be appreciated in the context of the present disclosure that a "beam" need not imply free-space optics but may also refer to light carried by waveguides such as fibre optics and other waveguides for carrying light.

As another example, some of lasers are mentioned above but the other types of suitable laser include a Distributed Bragg Reflector laser ('DBR'), a Fourier Domain Mode Locking laser ('FDML'), a Vertical Cavity Surface-Emitting laser ('VCSEL'). Additionally, or alternatively, a pulsed supercontinuum laser may be used in combination with a pulse stretching mechanism, such as a grating or GRISM pulse stretcher or length of dispersive optical fibre. For example, such an arrangement may be configured to temporally separate the wavelengths in the pulse such that a frequency chirped pulse is created (e.g., for ultimately providing an interferogram when sample and reference pulses are compared). Additionally, or alternatively, the wavelength sweeping may be provided by using a high coherence laser such as an external cavity diode laser (ECDL), DBR laser, feedback-stabilised laser or line-locked laser. Irrespective of the precise light source chosen, it typically is configured to provide wavelength swept light. This may be provided as a series of pulses. During each pulse, the wavelength may be "swept" through a range, which may provide a chirped pulse.

Any feature of any one of the examples disclosed herein may be combined with any selected features of any of the other examples described herein. For example, features of methods may be implemented in suitably configured hardware, and the configuration of the specific hardware described herein may be employed in methods implemented using other hardware.

It will be appreciated from the discussion above that the embodiments shown in the Figures are merely exemplary, and include features which may be generalised, removed or replaced as described herein and as set out in the claims.

With reference to the drawings in general, it will be appreciated that schematic functional block diagrams are used to indicate functionality of systems and apparatus described herein. It will be appreciated however that the functionality need not be divided in this way, and should not be taken to imply any particular structure of hardware other than that described and claimed below. The function of one or more of the elements shown in the drawings may be further subdivided, and/or distributed throughout apparatus of the disclosure. In some embodiments the function of one or more elements shown in the drawings may be integrated into a single functional unit. For example, the light combiner and the detector have been described as separate entities, but the two may be integrated so that the delayed reference light and the sample output light can be provided to a single device which both combines the two and detects the resultant light signal.

In some examples the functionality of the controller may be provided by a general purpose processor, which may be configured to perform a method according to any one of those described herein. In some examples the controller may comprise digital logic, such as field programmable gate arrays, FPGA, application specific integrated circuits, ASIC, a digital signal processor, DSP, or by any other appropriate hardware. In some examples, one or more memory elements can store data and/or program instructions used to implement the operations described herein. Embodiments of the disclosure provide tangible, non-transitory storage media comprising program instructions operable to program a processor to perform any one or more of the methods described and/or claimed herein and/or to provide data processing apparatus as described and/or claimed herein. The controller may comprise an analogue control circuit which provides at least a part of this control functionality. An embodiment provides an analogue control circuit configured to perform any one or more of the methods described herein.

The above embodiments are to be understood as illustrative examples. Further embodiments are envisaged. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

**Claims**

1.  An apparatus comprising:

    a reference channel for providing a reference light from a wavelength swept light source to a light combiner wherein the reference channel is configured to delay the reference light by a selected delay;
    a sample input channel for providing a sample input light from said wavelength swept light source and for directing the sample input light into a sample;
    a sample output channel for collecting, from the sample, a sample output light based on said sample input light and comprising a plurality of components each delayed, with respect to the sample input light, by a respective one of a plurality of delays;
    wherein the light combiner is configured to combine said sample output light with said delayed reference light to provide a combined light comprising a plurality of beat frequency components, each beat frequency component associated with a respective corresponding one of the plurality of delays; and,
    a controller configured with a predefined relationship for determining, from frequencies of the beat frequency components, a distribution of times-of-flight of light through the sample;
    wherein the predefined relationship is based on the selected delay provided by the reference channel being longer than any of the plurality of delays.

2.  The apparatus of claim 1 wherein the predefined relationship is a monotonically decreasing relationship that associates beat frequency components of lower frequency with longer times-of-flight and beat frequency components of higher frequency with shorter times-of-flight.

3.  The apparatus of claim 1 or 2 further comprising the wavelength swept light source.

4.  The apparatus of any preceding claim comprising a light splitter configured to split light from said wavelength swept light source to provide the reference light and the sample input light.

5.  The apparatus of any preceding claim comprising a variable delay line between one of:

    (i) said light source and said sample and
    (ii) said sample and said light combiner.

6.  The apparatus of claim 5 wherein the controller is configured to control the variable delay line to adjust said delays of said sample output light, wherein the adjusting is based on frequencies of the beat frequency components.

7.  The apparatus of any preceding claim further comprising a variable delay line in said reference channel.

8.  The apparatus of claim 7 wherein the controller is configured to control the variable delay line to adjust the selected delay based on frequencies of the beat frequency components.

9.  A computer implemented data processing method for determining times-of-flight of light through a sample up to a maximum time-of-flight associated with a maximum delay of interest, the method comprising:

obtaining input data from a light detector, wherein the input data defines a combined light signal obtained from interference of a sample output light with a delayed reference light;

determining, from the input data, frequency data defining beat frequencies of components of the input data;

determining a plurality of times-of-flight from the frequency data based on a predefined relationship associating lower beat frequencies with longer times-of-flight through the sample and higher beat frequencies with shorter times-of-flight through the sample;

wherein the sample output light comprises a plurality of light components collected from a sample and based on sample input light provided into the sample; and

wherein the delayed reference light corresponds to the sample input light delayed by a selected delay greater than the maximum delay of interest.

10. The computer implemented method of claim 9 wherein the predefined relationship is based on the selected delay provided by the reference channel being longer than any of the plurality of delays.

11. The computer implemented method of claim 10 wherein the predefined relationship is a monotonically decreasing relationship that associates beat frequency components of lower frequency with longer times-of-flight and beat frequency components of higher frequency with shorter times-of-flight.

12. A method comprising the computer implemented method of claim 9, 10, or 11 and further comprising operating the light detector to detect the combined light signal.

13. The method of claim 12 further comprising operating a wavelength swept light source of a spectrographic measurement system to provide the reference light and the sample input light.

14. The method of claim 12 or 13 further comprising controlling at least one variable delay line to provide an adjustable delay of at least one of the reference light and the sample input light, for example wherein the adjustable delay is determined adaptively based on frequencies of the beat frequency components.

15. A computer program product configured to program a programmable processor to perform the method of any of claims 9 to 14.

Figure. 1

y

Figure. 2

```
                    ( ------ )
                        |
                        v
       +-----------------------------------+
       |  Provide reference light and      |   100
       |  sample input light               |
       +-----------------------------------+
                        |
                        v
       +-----------------------------------+
       |  Direct the sample input light    |   110
       |  into a sample                    |
       +-----------------------------------+
                        |
                        v
       +-----------------------------------+
       |  Collect sample output light      |   120
       |  from the sample                  |
       +-----------------------------------+
                        |
                        v
       +-----------------------------------+
       |  delay reference light by a       |   140
       |  delay greater than the maximum   |
       |  delay of interest                |
       +-----------------------------------+
                        |
                        v
       +-----------------------------------+
       |  combine sample output light      |   150
       |  with delayed reference light     |
       +-----------------------------------+
                        |
                        v
       +-----------------------------------+
       |  determine times of flight        |   160
       +-----------------------------------+
                        |
                        v
                    ( ------ )
```

Figure. 3

160

Obtain input data — 1601

Determine, from the input data, frequency data — 1602

Determine times of flight from the frequency data — 1603

Figure. 4

```
        ╭────────╮
        ╰───┬────╯
            │
            ▼
   ┌─────────────────────┐
   │ Position probes on  │         ⌇ 200
   │       sample        │
   └──────────┬──────────┘
              │
              ▼
   ┌─────────────────────┐
   │ Obtain combined     │
   │ light signal and    │
   │ determine TOF       │          202
   │ distribution from   │
   │ combined light      │
   │ signal              │
   └──────────┬──────────┘
              │
              ▼
        ┌──────────┐         204
        │ Adjust   │
        │ delay    │
        └────┬─────┘
             │
             ▼
 ┌──────────────────────────────────────┐        206
 │ Obtain combined light signal and     │
 │ determine TOF distribution from      │
 │ combined light signal                │
 └──────────────────┬───────────────────┘
                    │
                    ▼
                 ◇◇◇◇◇◇
```

is maximum delay of interest at lower frequency than shorter delays?   208

Adjust delay   210

End set-up – apparatus is configured for measurement   212

Figure. 5

5A

5B

5C

5D

Figure. 6

```
                    ┌──────────────┐
                    │              │
                    └──────────────┘
                           │
              ┌────────────────────────┐
              │    Position probes     │
              │ on test material of ╱──┼──~  300
              │     known path         │
              │        length          │
              └────────────────────────┘
                           │
          ┌──────────────────────────────┐
          │                              │
          │ Obtain combined light signal │ ~  302
          │                              │
          └──────────────────────────────┘
                           │
          ┌──────────────────────────────┐
          │  Perform frequency transform │
          │   of combined light signal   │ ~  304
          │                              │
          └──────────────────────────────┘
                           │
              ┌────────────────────────┐
              │   Determine delay      │
              │     of delayed         │ ~  306
              │  reference light       │
              └────────────────────────┘
                           │
                    ┌──────────────┐
                    │              │
                    └──────────────┘
```

Europäisches
Patentamt

European
Patent Office

Office européen
des brevets

## EUROPEAN SEARCH REPORT

**Application Number**

EP 24 20 5987

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/023846 A1 (YANG WEIJIAN [US] ET AL) 25 January 2024 (2024-01-25) * abt; paragraph [0063] - paragraph [0175]; figures 1-12 * | 1-15 | INV. A61B5/00 G01B9/02004 G01B9/02 G01B9/02055 G01B9/0209 |
| X | GB 2 621 993 A (COMIND TECH LIMITED [GB]) 6 March 2024 (2024-03-06) * abstract; the whole document * | 1-15 | G01B9/02091 G01N21/47 |
| X | US 11 096 585 B2 (HI LLC [US]) 24 August 2021 (2021-08-24) * abstract; column 4, line 60; figures 1-8 * | 1-15 | |
| A | US 2020/060542 A1 (ALFORD JAMU [US] ET AL) 27 February 2020 (2020-02-27) * the whole document * | 1-15 | |
| A | OYBEK KHOLIQOV ET AL: "Interferometric near-infrared spectroscopy (iNIRS): performance tradeoffs and optimization", OPTICS EXPRESS, vol. 25, no. 23, 3 November 2017 (2017-11-03), page 28567, XP055643885, DOI: 10.1364/OE.25.028567 * the whole document * | 1-15 | **TECHNICAL FIELDS SEARCHED (IPC)** A61B G01B G01N |

The present search report has been drawn up for all claims

| Place of search | Date of completion of the search | Examiner |
|---|---|---|
| The Hague | 3 February 2025 | Munteh, Louis |

EPO FORM 1503 03.82 (P04C01)

## ANNEX TO THE EUROPEAN SEARCH REPORT
## ON EUROPEAN PATENT APPLICATION NO.

EP 24 20 5987

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2025

| Patent document cited in search report | | Publication date | Patent family member(s) | | Publication date |
|---|---|---|---|---|---|
| US 2024023846 | A1 | 25-01-2024 | US | 2024023846 A1 | 25-01-2024 |
| | | | WO | 2022115643 A1 | 02-06-2022 |
| GB 2621993 | A | 06-03-2024 | GB | 2621993 A | 06-03-2024 |
| | | | WO | 2024042339 A1 | 29-02-2024 |
| US 11096585 | B2 | 24-08-2021 | NONE | | |
| US 2020060542 | A1 | 27-02-2020 | US | 2020060542 A1 | 27-02-2020 |
| | | | US | 2023301515 A1 | 28-09-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2619063 A